# EUROPEAN PATENT APPLICATION

(11) **EP 1 262 552 A1**
(43) Date of publication of application: **04.12.2002**
(21) Application number: 02011349.4
(22) Date of filing: 23.05.2002
(51) Int. Cl.: C12N 15/54, C12N 15/62, C12N 15/11, C12N 9/10, C12N 1/21, G01N 33/68, A01N 63/00

(54) **Polynucleotides encoding insect glutaminyl cyclase and uses thereof**

(30) Priority: 23.05.2001 US 293369 P
(71) Applicant: Genoptera, LLC, South San Francisco, CA 94083-0511 (US)
(72) Inventor: Ebens, Allen James jr., San Francisco CA 94122 (US); Breach, Jean Claude R., San Francisco CA 94103 (US); Johnston, Stuart, Menlo Park CA 94025 (US); Mazzotta, Julie B., Novato CA 94945 (US)
(74) Representative: Grünecker, Kinkeldey, Stockmair & Schwanhäusser Anwaltssozietät

(57) **Abstract**

The invention provides polynucleotides encoding insect glutaminyl cyclase, as well as polypeptides encoded thereby. The invention further provides host cells comprising expression vectors comprising polynucleotides of the invention. Isolated polypeptides and host cells of the invention are useful in methods of screening for agents that reduce glutaminyl cyclase activity. Such agents are useful as pesticides.

## Description

### FIELD OF THE INVENTION

The invention relates to insect enzymes, and in particular to an insect glutaminyl cyclase.

### BACKGROUND OF THE INVENTION

Glutaminyl cyclase (QC) acts on amino-terminal glutamine residues of secreted proteins to cyclize glutamine resulting in release of ammonia and formation of a peptidyl-pyroglutaminyl residue. This modification is known to be essential for the activity of human neuropeptides such as thyrotropin releasing hormone (TRH) and gonadotropin releasing hormone (GnRH). Insect neuropeptides such as the sulfakinins are also known to be cyclized at N-terminal glutamine residues.

Pesticide development has traditionally focused on the chemical and physical properties of the pesticide itself, a relatively time-consuming and expensive process. As a consequence, efforts have been concentrated on the modification of pre-existing, well-validated compounds, rather than on the development of new pesticides. There is a need in the art for new pesticidal compounds that are safer, more selective, and more efficient than currently available pesticides. The present invention addresses this need by providing novel pesticide targets from invertebrates such as the tobacco budworm *Heliothis virescens,* and by providing methods of identifying compounds that bind to and modulate the activity of such targets.

### Literature

Predel et al. Post-translational modifications of the insect sulfakinins: sulfation, pyroglutamate-formation and O-methylation of glutamic acid. (1999) *Eur J Biochem* 263(2):552-60; Gololobov et al. (1996) *Biol. Chem. Hoppe Seyler* 377(6):395-398; Song et al. (1994) *J*. *Mol. Endocrinol.* 13(1):77-86; Bateman (1989) *J*. *Neurosci. Methods* 30(1):23-28; and Busby et al. (1987) *J*. *Biol. Chem.* 262(18):8532-8536.

### SUMMARY OF THE INVENTION

It is an object of the invention to provide isolated insect nucleic acid molecules and proteins that are targets for pesticides. The isolated insect nucleic acid molecules provided herein are useful for producing insect proteins encoded thereby. The insect proteins are useful in assays to identify compounds that modulate a biological activity of the proteins, which assays identify compounds that may have utility as pesticides. It is an object of the present invention to provide invertebrate genes encoding enzymes that can be used in genetic screening methods to characterize pathways that such genes may be involved in, as well as other interacting genetic pathways. It is also an object of the invention to provide methods for screening compounds that interact with a subject invertebrate enzyme. Compounds that interact with a subject invertebrate enzyme may have utility as therapeutics or pesticides.

### BRIEF DESCRIPTION OF THE DRAWINGS

Figure 1 provides the amino acid sequence of a *Heliothis* glutaminyl cyclase (SEQ ID NO:02).

Figure 2 provides the nucleotide sequence of a *Heliothis* glutaminyl cyclase cDNA (SEQ ID NO:01).

### DEFINITIONS

As used herein the term "isolated" is meant to describe a polynucleotide, a polypeptide, an antibody, or a host cell that is in an environment different from that in which the polynucleotide, the polypeptide, the antibody, or the host cell naturally occurs. As used herein, the term "substantially purified" refers to a compound (e.g., either a polynucleotide or a polypeptide or an antibody) that is removed from its natural environment and is at least 60% free, at least 75% free, or at least 90% free from other components with which it is naturally associated.

The terms "polynucleotide" and "nucleic acid molecule", used interchangeably herein, refer to a polymeric forms of nucleotides of any length, either ribonucleotides or deoxynucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidites and thus can be an oligodeoxynucleoside phosphoramidate or a mixed phosphoramidate-phosphodiester oligomer. Peyrottes et al. (1996) *Nucl. Acids Res.* 24:1841-1848; Chaturvedi et al. (1996) *Nucl. Acids Res.* 24:2318-2323. A polynucleotide may comprise modified nucleotides, such as methylated nucleotides and nucleotide analogs, uracyl, other sugars, and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support.

For hybridization probes, it may be desirable to use nucleic acid analogs, in order to improve the stability and binding affinity. A number of modifications have been described that alter the chemistry of the phosphodiester backbone, sugars or heterocyclic bases.

Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O-5'-S-phosphorothioate, 3'-S-5'-O-phosphorothioate, 3'-CH2-5'-O-phosphonate and 3'-NH-5'-O-phosphoroamidate. Peptide nucleic acids replace the entire phosphodiester backbone with a peptide linkage.

Sugar modifications are also used to enhance stability and affinity. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without compromising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

The terms "polypeptide" and "protein", used interchangeably herein, refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

A "host cell", as used herein, denotes microorganisms or eukaryotic cells or cell lines cultured as unicellular entities which can be, or have been, used as recipients for recombinant vectors or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation. A "recombinant host cell" is a host cell into which has been introduced a subject nucleic acid molecule or a subject recombinant vector.

By "transformation" is meant a permanent or transient genetic change induced in a cell following incorporation of new DNA (i.e., DNA exogenous to the cell). Genetic change can be accomplished either by incorporation of the new DNA into the genome of the host cell, or by transient or stable maintenance of the new DNA as an episomal element. Where the cell is a eukaryotic cell, a permanent genetic change is generally achieved by introduction of the DNA into the genome of the cell.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a pesticidal agent" includes a plurality of such agents and reference to "the glutaminyl cyclase" includes reference to one or more glutaminyl cyclase and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION OF THE INVENTION

### ISOLATED NUCLEIC ACID MOLECULES OF THE INVENTION

The invention provides isolated insect nucleic acid molecules comprising nucleotide sequences of insect glutaminyl cyclase, particularly nucleic acid sequences of Lepidopteran glutaminyl cyclase, and more particularly nucleic acid sequences of *Heliothis virescens* glutaminyl cyclase, and methods of using these nucleic acid molecules.

The present invention provides isolated nucleic acid molecules that comprise nucleotide sequences encoding insect proteins that are potential pesticide targets. The isolated nucleic acid molecules have a variety of uses, e.g., as hybridization probes, e.g., to identify nucleic acid molecules that share nucleotide sequence identity; in expression vectors to produce the polypeptides encoded by the nucleic acid molecules; and to modify a host cell or animal for use in assays described hereinbelow.

The term "isolated nucleic acid sequence", as used herein, includes the reverse complement, RNA equivalent, DNA or RNA single- or double-stranded sequences, and DNA/RNA hybrids of the sequence being described, unless otherwise indicated.

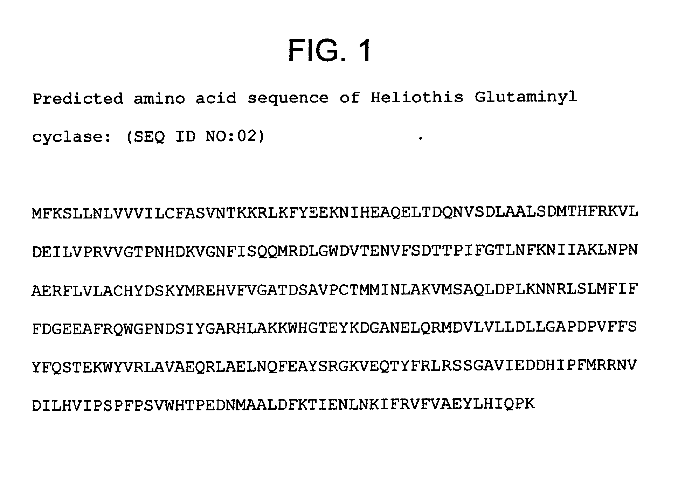

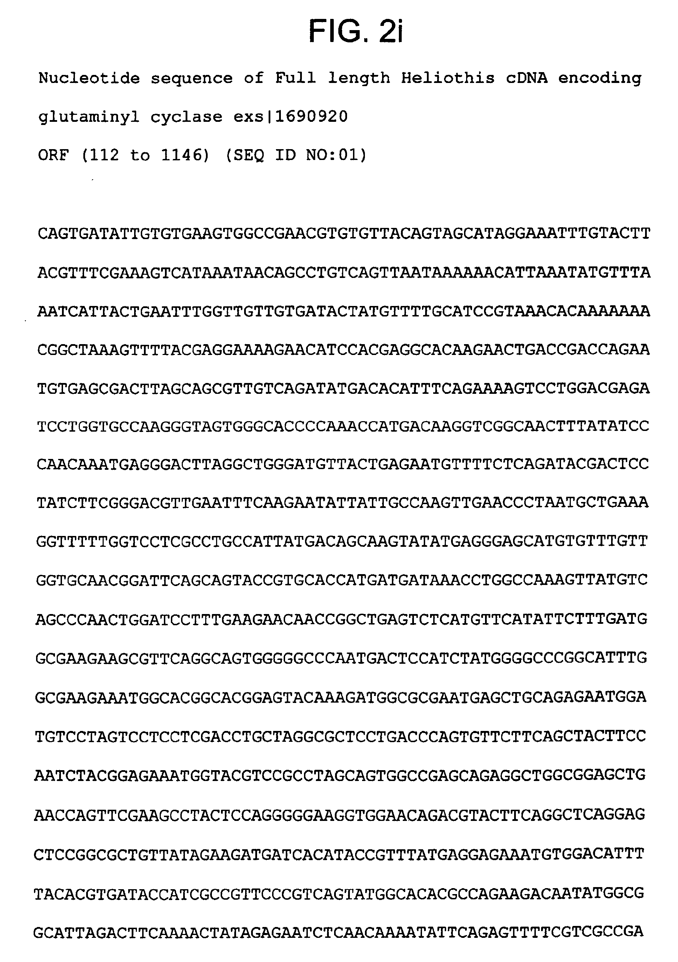

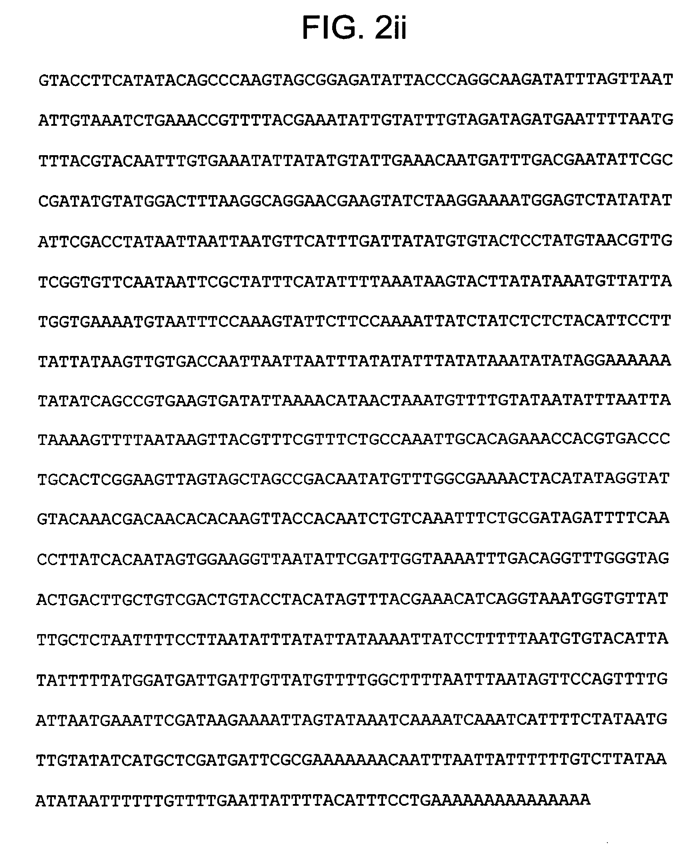

Figures 1 and 2 provide the amino acid (SEQ ID NO:02) and nucleotide (SEQ ID NO:01) sequences, respectively, of a glutaminyl cyclase from *Heliothis.* Nucleotides 112 to 1146 of SEQ ID NO:01 represent the coding sequences that encode the amino acid sequence of SEQ ID NO:02.

In some embodiments, an insect glutaminyl cyclase nucleic acid molecule comprises a nucleotide sequence having at least about 50%, at least about 60%, at least about 65%, at least about 70%, at least about 75%, at least about 80%, at least about 85%, or at least about 90%, or more, nucleotide sequence identity with the sequence set forth in SEQ ID NO:01. In other embodiments, an insect glutaminyl cyclase nucleic acid molecule comprises a nucleotide sequence having the sequence set forth in SEQ ID NO:01.

In some embodiments, an insect glutaminyl cyclase nucleic acid molecule comprises a nucleotide sequence having at least about 50%, at least about 60%, at least about 65%, at least about 70%, at least about 75%, at least about 80%, at least about 85%, or at least about 90%, or more, nucleotide sequence identity with the sequence set forth in nucleotides 112 to 1146 of SEQ ID NO:01. In other embodiments, an insect glutaminyl cyclase nucleic acid molecule comprises a nucleotide sequence having the sequence set forth in nucleotides 112 to 1146 of SEQ ID NO:01.

In other embodiments, an insect glutaminyl cyclase nucleic acid molecule comprises a fragment of at least about 18, at least about 25, at least about 30, at least about 35, at least about 40, at least about 50, at least about 75, at least about 100, at least about 125, at least about 150, at least about 200, at least about 250, at least about 300, at least about 350, at least about 400, at least about 450, at least about 500, at least about 550, at least about 600, at least about 650, at least about 700, at least about 750, at least about 800, at least about 850, at least about 900, at least about 950, or at least about 1000, contiguous nucleotides of nucleotides 112-1146 of the sequence set forth in SEQ ID NO:01.

In other embodiments, an insect glutaminyl cyclase nucleic acid molecule comprises a fragment of at least about 18, at least about 25, at least about 30, at least about 35, at least about 40, at least about 50, at least about 75, at least about 100, at least about 125, at least about 150, at least about 200, at least about 250, at least about 300, at least about 350, at least about 400, at least about 450, at least about 500, at least about 550, at least about 600, at least about 650, at least about 700, at least about 750, at least about 800, at least about 850, at least about 900, at least about 950, at least about 1000, at least about 1100, at least about 1200, at least about 1300, at least about 1400, at least about 1500, at least about 1600, at least about 1700, at least about 1800, at least about 1900, at least about 2000, at least about 2100, or at least about 2200, contiguous nucleotides of the sequence set forth in SEQ ID NO:01.

In other embodiments, an insect glutaminyl cyclase nucleic acid molecule comprises a nucleotide sequence encoding a polypeptide comprising an amino acid sequence having at least about 60%, at least about 65%, at least about 70%, at least about 75%, at least about 80%, at least about 85%, at least about 90%, or at least about 95%, amino acid sequence identity with the amino acid sequence set forth in SEQ ID NO:02. In some embodiments, an insect glutaminyl cyclase nucleic acid molecule comprises a nucleotide sequence encoding a polypeptide comprising the sequence set forth in SEQ ID NO:02. In many of these embodiments, the encoded polypeptide has glutaminyl cyclase activity.

In other embodiments, an insect glutaminyl cyclase nucleic acid molecule comprises a nucleotide sequence encoding a polypeptide comprising a fragment of at least about 6, at least about 10, at least about 15, at least about 20, at least about 25, at least about 30, at least about 40, at least about 50, at least about 75, at least about 100, at least about 125, at least about 150, at least about 175, at least about 200, at least about 225, at least about 250, at least about 275, at least about 300, at least about 325, or at least about 340, contiguous amino acids of the sequence set forth in SEQ ID NO:02. In many of these embodiments, the encoded polypeptide has glutaminyl cyclase activity.

Fragments of the subject nucleic acid molecules can be used for a variety of purposes. Interfering RNA (RNAi) fragments, particularly double-stranded (ds) RNAi, can be used to generate loss-of-function phenotypes, or to formulate biopesticides (discussed further below). The subject nucleic acid fragments are also useful as nucleic acid hybridization probes and replication/amplification primers. Certain "antisense" fragments, i.e. that are reverse complements of portions of the coding sequence of SEQ ID NO:01 have utility in inhibiting the function of a subject protein. The fragments are of length sufficient to specifically hybridize with a nucleic acid molecule having the sequence set forth in SEQ ID NO:01. The fragments consist of or comprise at least 12, at least 24, at least 36, or at least 96 contiguous nucleotides of SEQ ID NO:01. When the fragments are flanked by other nucleic acid sequences, the total length of the combined nucleic acid sequence is less than 15 kb, less than 10 kb or less than 5kb, or less than 2 kb.

The subject nucleic acid sequences may consist solely of SEQ ID NO:01 or fragments thereof. Alternatively, the subject nucleic acid sequences and fragments thereof may be joined to other components such as labels, peptides, agents that facilitate transport across cell membranes, hybridization-triggered cleavage agents or intercalating agents. The subject nucleic acid sequences and fragments thereof may also be joined to other nucleic acid sequences (i.e. they may comprise part of larger sequences) and are of synthetic/non-natural sequences and/or are isolated and/or are purified, i.e. unaccompanied by at least some of the material with which it is associated in its natural state. In many embodiments, the isolated nucleic acids constitute at least about 0.5%, or at least about 5% by weight of the total nucleic acid present in a given fraction, and are generally recombinant, meaning that they comprise a non-natural sequence or a natural sequence joined to nucleotide(s) other than that which it is joined to on a natural chromosome.

Derivative nucleic acid molecules of the subject nucleic acid molecules include sequences that hybridize to the nucleic acid sequence of SEQ ID NO:01, or to a nucleic acid molecule containing the open reading frame of SEQ ID NO:01, under stringency conditions such that the hybridizing derivative nucleic acid is related to the subject nucleic acid by a certain degree of sequence identity. A nucleic acid molecule is "hybridizable" to another nucleic acid molecule, such as a cDNA, genomic DNA, or RNA, when a single stranded form of the nucleic acid molecule can anneal to the other nucleic acid molecule. Stringency of hybridization refers to conditions under which nucleic acids are hybridizable. The degree of stringency can be controlled by temperature, ionic strength, pH, and the presence of denaturing agents such as formamide during hybridization and washing. As used herein, the term "stringent hybridization conditions" are those normally used by one of skill in the art to establish at least a 90% sequence identity between complementary pieces of DNA or DNA and RNA. "Moderately stringent hybridization conditions" are used to find derivatives having at least 70% sequence identity. Finally, "low-stringency hybridization conditions" are used to isolate derivative nucleic acid molecules that share at least about 50% sequence identity with the subject nucleic acid sequence.

The ultimate hybridization stringency reflects both the actual hybridization conditions as well as the washing conditions following the hybridization, and it is well known in the art how to vary the conditions to obtain the desired result. Conditions routinely used are set out in readily available procedure texts (*e.g.*, Current Protocol in Molecular Biology, Vol. 1, Chap. 2.10, John Wiley & Sons, Publishers (1994); Sambrook *et al.,* Molecular Cloning, Cold Spring Harbor (1989)). In some embodiments, a nucleic acid molecule of the invention is capable of hybridizing to a nucleic acid molecule containing a nucleotide sequence as set forth in SEQ ID NO:01 under stringent hybridization conditions that comprise: prehybridization of filters containing nucleic acid for 8 hours to overnight at 65° C in a solution comprising 6X single strength citrate (SSC) (1X SSC is 0.15 M NaCl, 0.015 M Na citrate; pH 7.0), 5X Denhardt's solution, 0.05% sodium pyrophosphate and 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1 h in a solution containing 0.2X SSC and 0.1% SDS (sodium dodecyl sulfate).

Derivative nucleic acid sequences that have at least about 70% sequence identity with SEQ ID NO:01 are capable of hybridizing to a nucleic acid molecule containing a nucleotide sequence as set forth in SEQ ID NO:1 under moderately stringent conditions that comprise: pretreatment of filters containing nucleic acid for 6 h at 40° C in a solution containing 35% formamide, 5X SSC, 50 mM Tris-HCI (pH 7.5), 5 mM EDTA, 0.1% PVP, 0.1% Ficoll, 1% BSA, and 500 µg/ml denatured salmon sperm DNA; hybridization for 18-20 h at 40° C in a solution containing 35% formamide, 5X SSC, 50 mM Tris-HCl (pH 7.5), 5 mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1% SDS.

Other exemplary derivative nucleic acid sequences are capable of hybridizing to SEQ ID NO:01 under low stringency conditions that comprise: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

As used herein, "percent (%) nucleic acid sequence identity" with respect to a subject sequence, or a specified portion of a subject sequence, is defined as the percentage of nucleotides in the candidate derivative nucleic acid sequence identical with the nucleotides in the subject sequence (or specified portion thereof), after aligning the sequences and introducing gaps, if necessary to achieve the maximum percent sequence identity, as generated by the program WU-BLAST-2.0a19 (Altschul *et al.,* J. Mol. Biol. (1997)215:403-410; http://blast.wustl.edu/blast/README.html; hereinafter referred to generally as "BLAST") with all the search parameters set to default values. The HSP S and HSP S2 parameters are dynamic values and are established by the program itself depending upon the composition of the particular sequence and composition of the particular database against which the sequence of interest is being searched. A percent (%) nucleic acid sequence identity value is determined by the number of matching identical nucleotides divided by the sequence length for which the percent identity is being reported.

In one embodiment, the derivative nucleic acid encodes a polypeptide comprising an amino acid sequence set forth in SEQ ID NO:02, or a fragment or derivative thereof as described further below. A derivative of a subject nucleic acid molecule, or fragment thereof, may comprise 100% sequence identity with SEQ ID NO:01, but be a derivative thereof in the sense that it has one or more modifications at the base or sugar moiety, or phosphate backbone. Examples of modifications are well known in the art (Bailey, Ullmann's Encyclopedia of Industrial Chemistry (1998), 6th ed. Wiley and Sons). Such derivatives may be used to provide modified stability or any other desired property.

As used herein, a "derivative" nucleic acid or amino acid sequence includes orthologous sequences of SEQ ID NO:01 and SEQ ID NO:02, that are derived from other species. In some embodiments, the orthologue is from a heliothine species, for example *Heliocoverpa armigera* and *Heliothis zea,* which, together with *Heliothis virescens* are three of the world's major crop pests. Orthologous genes of these three species are extremely similar (The International Meeting on Genomics of Lepidoptera, Lyon, France August 16-17, 2001; "International Lepidopteran Genome Project Proposal," Rev. September 10, 2001; available at world wide web site ab.a.u-tokyo.ac.jp/lep-genome/.

In another example, it may be desired to develop a pesticidal agent that specifically targets a non-Heliothine insect species. In such case, it may be most efficient to develop biochemical screening assays (*i.e.,* assays designed to identify molecules that can inhibit the protein target, as described hereinbelow) using the orthologous protein from that insect. While the orthologues in two species may have essentially the same function, differences in their protein structure may affect properties such as interactions with other proteins, compound binding and stability. Thus, results of a biochemical assays are most meaningful for the specific protein used in the assay. As used herein, orthologues include nucleic acid and polypeptide sequences.

Methods of identifying the orthologues in other species are known in the art. Normally, orthologues in different species retain the same function, due to presence of one or more protein motifs and/or 3-dimensional structures. In evolution, when a gene duplication event follows speciation, a single gene in one species, such as *Heliothis,* may correspond to multiple genes (paralogs) in another. As used herein, the term "orthologues" encompasses paralogs. When sequence data is available for a particular species, orthologues are generally identified by sequence homology analysis, such as BLAST analysis, usually using protein bait sequences. Sequences are assigned as a potential orthologue if the best hit sequence from the forward BLAST result retrieves the original query sequence in the reverse BLAST (Huynen MA and Bork P, Proc Natl Acad Sci (1998) 95:5849-5856; Huynen MA *et al.,* Genome Research (2000) 10:1204-1210). Programs for multiple sequence alignment, such as CLUSTAL-W (Thompson JD et al, 1994, Nucleic Acids Res 22:4673-4680) may be used to highlight conserved regions and/or residues of orthologous proteins and to generate phylogenetic trees. In a phylogenetic tree representing multiple homologous sequences from diverse species (e.g., retrieved through BLAST analysis), orthologous sequences from two species generally appear closest on the tree with respect to all other sequences from these two species.

Structural threading or other analysis of protein folding (e.g., using software by ProCeryon, Biosciences, Salzburg, Austria) may also identify potential orthologues. Nucleic acid hybridization methods may also be used to find orthologous genes, e.g., when sequence data are not available. Degenerate PCR and screening of cDNA or genomic DNA libraries are common methods for finding related gene sequences and are well known in the art (see, e.g., Sambrook *et al.* Molecular Cloning: A Laboratory Manual (Second Edition), Cold Spring Harbor Press, Plainview, N.Y., 1989; Dieffenbach C and Dveksler G (Eds.) PCR Primer: A Laboratory Manual, Cold Spring Harbor Laboratory Press, NY, 1989). For instance, methods for generating a cDNA library from an insect species of interest and probing the library with partially homologous gene probes are described in Sambrook *et al.* A highly conserved portion of the *Heliothis* glutaminyl cyclase coding sequence may be used as a probe. Glutaminyl cyclase orthologue nucleic acids may hybridize to the nucleic acid of SEQ ID NO: 1 under high, moderate, or low stringency conditions.

After amplification or isolation of a segment of a putative orthologue, that segment may be cloned and sequenced by standard techniques and utilized as a probe to isolate a complete cDNA or genomic clone. Alternatively, it is possible to initiate an EST project to generate a database of sequence information for the species of interest. In another approach, antibodies that specifically bind known glutaminyl cyclase polypeptides are used for orthologue isolation (Harlow E and Lane D, Antibodies: A Laboratory Manual, Cold Spring Harbor Laboratory Press, 1988, New York; Harlow E and Lane D, Using Antibodies: A Laboratory Manual, Cold Spring Harbor Laboratory Press, 1999, New York).

Western blot analysis can determine that a glutaminyl cyclase orthologue (i.e., an orthologous protein) is present in a crude extract of tissue from a particular species. When reactivity is observed, the sequence encoding the candidate orthologue may be isolated by screening expression libraries representing the particular species. Expression libraries can be constructed in a variety of commercially available vectors, including lambda gt11, as described in Sambrook, *et al.* Once the candidate orthologue(s) are identified by any of these means, candidate orthologous sequence are used as bait (the "query") for the reverse BLAST against sequences from *Heliothis* or other species in which glutaminyl cyclase nucleic acid and/or polypeptide sequences have been identified.

Another type of derivative of the subject nucleic acid sequences includes corresponding humanized sequences. A humanized nucleic acid sequence is one in which one or more codons has been substituted with a codon that is more commonly used in human genes. Generally, a sufficient number of codons have been substituted such that a higher level expression is achieved in mammalian cells than what would otherwise be achieved without the substitutions. Tables are available in the art that show, for each amino acid, the calculated codon frequency in humans genes for 1000 codons (Wada *et al*., Nucleic Acids Research (1990) 18(Suppl.):2367-2411). Similarly, other nucleic acid derivatives can be generated with codon usage optimized for expression in other organisms, such as yeasts, bacteria, and plants, where it is desired to engineer the expression of receptor proteins by using specific codons chosen according to the preferred codons used in highly expressed genes in each organism. Thus, a subject nucleic acid molecule in which the glutamic acid codon, GAA has been replaced with the codon GAG, which is more commonly used in human genes, is an example of a humanized nucleic acid molecule. A detailed discussion of the humanization of nucleic acid sequences is provided in U.S. Pat. No. 5,874,304 to Zolotukhin *et al.*

### Isolation, Production, and Expression of Subject Nucleic Acid Molecules

The subject nucleic acid molecules, or fragments or derivatives thereof, may be obtained from an appropriate cDNA library prepared from any suitable insect species (including, but not limited to, *Drosophila.* and *Heliothis*). In many embodiments, a lepidopteran species is used, e.g., a heliothine species. Where the subject nucleic acid molecule is isolated from a Heliothine species, any of a variety of field and laboratory strains of various *Heliothis* species can be used, including, but not limited to, *Heliothis virescens, Heliothis maritima, Heliothis ononis, Heliothis peltigera, Heliothis phloxiphaga, Helicoverpa punctigera, Heliothis subflexa, Helicoverpa armigera,* and *Helicoverpa zea.*

An expression library can be constructed using known methods. For example, mRNA can be isolated to make cDNA which is ligated into a suitable expression vector for expression in a host cell into which it is introduced. Various screening assays can then be used to select for the gene or gene product (*e.g.* oligonucleotides of at least about 20 to 80 bases designed to identify the gene of interest, or labeled antibodies that specifically bind to the gene product). The gene and/or gene product can then be recovered from the host cell using known techniques.

A polymerase chain reaction (PCR) can also be used to isolate a subject nucleic acid molecule, where oligonucleotide primers representing fragmentary sequences of interest amplify RNA or DNA sequences from a source such as a genomic or cDNA library (as described by Sambrook *et al*., *supra*). Additionally, degenerate primers for amplifying homologs from any species of interest may be used. Once a PCR product of appropriate size and sequence is obtained, it may be cloned and sequenced by standard techniques, and utilized as a probe to isolate a complete cDNA or genomic clone.

Fragmentary sequences of the subject nucleic acid molecules and derivatives thereof may be synthesized by known methods. For example, oligonucleotides may be synthesized using an automated DNA synthesizer available from commercial suppliers (*e.g.* Biosearch, Novato, CA; Perkin-Elmer Applied Biosystems, Foster City, CA). Antisense RNA sequences can be produced intracellularly by transcription from an exogenous sequence, *e.g.* from vectors that contain subject antisense nucleic acid sequences. Newly generated sequences may be identified and isolated using standard methods.

An isolated subject nucleic acid molecule can be inserted into any appropriate cloning vector, for example bacteriophages such as lambda derivatives, or plasmids such as pBR322, pUC plasmid derivatives and the Bluescript vector (Stratagene, San Diego, CA). Recombinant molecules can be introduced into host cells via transformation, transfection, infection, electroporation, *etc.,* or into a transgenic animal such as a fly. The transformed cells can be cultured to generate large quantities of the subject nucleic acid. Suitable methods for isolating and producing the subject nucleic acid sequences are well known in the art (Sambrook *et al., supra*; DNA Cloning: A Practical Approach, Vol. 1, 2, 3, 4, (1995) Glover, ed., MRL Press, Ltd., Oxford, U.K.).

The nucleotide sequence encoding a subject protein or fragment or derivative thereof, can be inserted into any appropriate expression vector for the transcription and translation of the inserted protein-coding sequence. Alternatively, the necessary transcriptional and translational signals can be supplied by the native subject gene and/or its flanking regions. A variety of host-vector systems may be utilized to express the protein-coding sequence such as mammalian cell systems infected with virus (*e.g.* vaccinia virus, adenovirus, *etc.*); insect cell systems infected with virus (*e.g.* baculovirus); microorganisms such as yeast containing yeast vectors, or bacteria transformed with bacteriophage, DNA, plasmid DNA, or cosmid DNA. Expression of a subject protein may be controlled by a suitable promoter/enhancer element. In addition, a host cell strain may be selected which modulates the expression of the inserted sequences, or modifies and processes the gene product in the specific fashion desired. Exemplary host cells include *E. coli,* lepidopteran Sf-9 or S-21 cells, and *Drosophila* S2 cells.

To detect expression of a subject gene product, the expression vector can comprise a promoter operably linked to a subject nucleic acid molecule, one or more origins of replication, and, one or more selectable markers (*e.g.* thymidine kinase activity, resistance to antibiotics, *etc.*). Alternatively, recombinant expression vectors can be identified by assaying for the expression of a subject gene product based on the physical or functional properties of a subject protein in *in vitro* assay systems (*e.g*. immunoassays).

A subject protein, fragment, or derivative may be optionally expressed as a fusion, or chimeric protein product (i.e. it is joined via a peptide bond to a heterologous protein sequence of a different, i.e., non-QC, protein). In one embodiment, the subject protein is expressed as a fusion protein with a "tag" that facilitates purification, such as glutathione-S-transferase or (His)₆. A chimeric product can be made by ligating the appropriate nucleic acid sequences encoding the desired amino acid sequences to each other in the proper coding frame using standard methods and expressing the chimeric product. A chimeric product may also be made by protein synthetic techniques, *e.g.* by use of a peptide synthesizer.

Once a recombinant vector that expresses a subject nucleic acid molecule is identified, the encoded QC polypeptide can be isolated and purified using standard methods (*e.g.* ion exchange, affinity, and gel exclusion chromatography; centrifugation; differential solubility; electrophoresis). The amino acid sequence of the protein can be deduced from the nucleotide sequence of the recombinant nucleic acid molecule contained in the recombinant vector and can thus be synthesized by standard chemical methods (Hunkapiller *et al.*, Nature (1984) 310:105-111). Alternatively, native subject proteins can be purified from natural sources, by standard methods (*e.g*. immunoaffinity purification).

### RECOMBINANT VECTORS AND HOST CELLS

Also provided are constructs ("recombinant vectors") comprising the subject nucleic acids inserted into a vector, and host cells comprising the constructs. The subject constructs are used for a number of different applications, including propagation, protein production, etc. Viral and non-viral vectors may be prepared and used, including plasmids. The choice of plasmid will depend on the type of cell in which propagation is desired and the purpose of propagation. Certain vectors are useful for amplifying and making large amounts of the desired DNA sequence. Other vectors are suitable for expression in cells in culture. Still other vectors are suitable for transfer and expression in cells in a whole animal. The choice of appropriate vector is well within the skill of the art. Many such vectors are available commercially.

To prepare the constructs, the partial or full-length polynucleotide is inserted into a vector typically by means of DNA ligase attachment to a cleaved restriction enzyme site in the vector. Alternatively, the desired nucleotide sequence can be inserted by homologous recombination *in vivo.* Typically this is accomplished by attaching regions of homology to the vector on the flanks of the desired nucleotide sequence. Regions of homology are added by ligation of oligonucleotides, or by polymerase chain reaction using primers comprising both the region of homology and a portion of the desired nucleotide sequence, for example.

Also provided are expression cassettes or systems that find use in, among other applications, the synthesis of the subject proteins. For expression, the gene product encoded by a polynucleotide of the invention is expressed in any convenient expression system, including, for example, bacterial, yeast, insect, amphibian, and mammalian systems. Suitable vectors and host cells are described in U.S. Patent No. 5,654,173. In the expression vector, a QC-encoding polynucleotide, e.g., as set forth in SEQ ID NO: 01, is operably linked to a regulatory sequence as appropriate to obtain the desired expression properties. These can include promoters (attached either at the 5' end of the sense strand or at the 3' end of the antisense strand), enhancers, terminators, operators, repressors, and inducers. The promoters can be regulated or constitutive. In some situations it may be desirable to use conditionally active promoters, such as tissue-specific, or developmental stage-specific promoters. These are linked to the desired nucleotide sequence using the techniques described above for linkage to vectors. Any techniques known in the art can be used. In other words, the expression vector will provide a transcriptional and translational initiation region, which may be inducible or constitutive, where the coding region is operably linked under the transcriptional control of the transcriptional initiation region, and a transcriptional and translational termination region. These control regions may be native to the subject QC gene, or may be derived from exogenous sources.

Expression vectors generally have convenient restriction sites located near the promoter sequence to provide for the insertion of nucleic acid sequences encoding heterologous proteins. A selectable marker operative in the expression host may be present, for detection of host cells that comprise the recombinant vector. A variety of markers are known and may be present on the vector, where such markers include those that confer antibiotic resistance, e.g. resistance to ampicillin, tetracycline, chloramphenicol, kanamycin (neomycin), markers that provide for histochemical detection, etc. Expression vectors may be used for, among other things, the production of QC proteins, QC fusion proteins, as described above, and for use in screening assays, as described below.

Expression cassettes may be prepared comprising a transcription initiation region, the gene or fragment thereof, and a transcriptional termination region. Of particular interest is the use of sequences that allow for the expression of functional epitopes or domains, usually at least about 8 amino acids in length, more usually at least about 15 amino acids in length, to about 25 amino acids, and up to the complete open reading frame of the gene. After introduction of the DNA, the cells containing the construct may be selected by means of a selectable marker, the cells expanded and then used for expression.

The above described expression systems may be employed with prokaryotes or eukaryotes in accordance with conventional ways, depending upon the purpose for expression. For large scale production of the protein, or for use in screening assays as described herein, a unicellular organism, such as *E. coli, B. subtilis, S. cerevisiae,* insect cells in combination with baculovirus vectors, or cells of a higher organism such as vertebrates, *e.g.* COS 7 cells, HEK 293, CHO, *Xenopus* oocytes, lepidopteran Sf-9 or S-21 cells, *Drosophila* S2 cells, may be used as the expression host cells. In some situations, it is desirable to express the gene in eukaryotic cells, where the expressed protein will benefit from native folding and post-translational modifications. Small peptides can also be synthesized in the laboratory. Polypeptides that are subsets of the complete protein sequence may be used to identify and investigate parts of the protein important for function.

Specific expression systems of interest include bacterial, yeast, insect cell and mammalian cell derived expression systems. Representative systems from each of these categories is are provided below:
Bacteria. Expression systems in bacteria include those described in Chang *et al.*, *Nature* (1978) 275:615; Goeddel *et al., Nature* (1979) *281*:544; Goeddel *et al., Nucleic Acids Res.* (1980) *8*:4057; EP 0 036,776; U.S. Patent No. 4,551,433; DeBoer *et al., Proc. Natl. Acad. Sci. (USA)* (1983) *80*:21-25; and Siebenlist *et al., Cell* (1980) 20:269.
Yeast. Expression systems in yeast include those described in Hinnen *et al., Proc. Natl. Acad. Sci. (USA)* (1978) *75*:1929; Ito *et al., J. Bacteriol.* (1983) *153*:163; Kurtz *et al., Mol. Cell. Biol.* (1986) *6*:142; Kunze *et al., J. Basic Microbiol.* (1985) *25*:141; Gleeson *et al., J. Gen. Microbiol.* (1986) *132*:3459; Roggenkamp *et al., Mol. Gen. Genet.* (1986) *202*:302; Das *et al., J. Bacteriol.* (1984) *158*:1165; De Louvencourt *et al., J. Bacteriol.* (1983) *154*:737; Van den Berg *et al., Bio*/*Technology* (1990) *8*:135; Kunze *et al., J. Basic Microbiol.* (1985) *25*:141; Cregg *et al., Mol. Cell. Biol.* (1985) *5*:3376; U.S. Patent Nos. 4,837,148 and 4,929,555; Beach and Nurse, *Nature* (1981) *300:706;* Davidow *et al., Curr. Genet.* (1985) *10*:380; Gaillardin *et al., Curr. Genet.* (1985) *10*:49; Ballance *et al., Biochem. Biophys. Res. Commun.* (1983) *112*:284-289; Tilburn *et al., Gene* (1983) *26*:205-221; Yelton *et al., Proc. Natl. Acad. Sci. (USA)* (1984) *81*:1470-1474; Kelly and Hynes, *EMBO J.* (1985) 4:475479; EP 0 244,234; and WO 91/00357.
Insect Cells. Expression of heterologous genes in insects is accomplished as described in U.S. Patent No. 4,745,051; Friesen *et al.,* "The Regulation of Baculovirus Gene Expression", in: *The Molecular Biology Of Baculoviruses* (1986) (W. Doerfler, ed.); EP 0 127,839; EP 0 155,476; and Vlak *et al., J. Gen. Virol.* (1988) *69*:765-776; Miller *et al., Ann. Rev. Microbiol.* (1988) *42*:177; Carbonell *et al., Gene* (1988) 73:409; Maeda *et al., Nature* (1985) *315*:592-594; Lebacq-Verheyden *et al., Mol. Cell. Biol.* (1988) *8*:3129; Smith *et al., Proc. Natl. Acad. Sci. (USA)* (1985) *82*:8844; Miyajima *et al., Gene* (1987) *58:273;* and Martin *et al., DNA* (1988) *7*:99. Numerous baculoviral strains and variants and corresponding permissive insect host cells from hosts are described in Luckow *et al., Bio*/*Technology* (1988) *6*:47-55, Miller *et al., Generic Engineering* (1986) *8*:277-279, and Maeda *et al., Nature* (1985) *315*:592-594. Various insect cells, including lepidopteran Sf-9 cells and S-21 cells, and *Drosophila* S2 cells, have been amply described in the art. See, e.g., "Insect Cell Culture Engineering", Goosen, Daugulis, and Faulkner, eds. (1993) Marcel Dekker.
Mammalian Cells. Mammalian expression is accomplished as described in Dijkema *et al., EMBO J.* (1985) *4*:761, Gorman *et al., Proc. Natl. Acad. Sci. (USA)* (1982) *79*:6777, Boshart *et al., Cell* (1985) *41*:521 and U.S. Patent No. 4,399,216. Other features of mammalian expression are facilitated as described in Ham and Wallace, *Meth. Enz.* (1979) *58*:44, Barnes and Sato, *Anal. Biochem.* (1980) *102*:255, U.S. Patent Nos. 4,767,704, 4,657,866, 4,927,762, 4,560,655, WO 90/103430, WO 87/00195, and U.S. RE 30,985.
Plant cells. Plant cell culture is amply described in various publications, including, e.g., Plant Cell Culture: A Practical Approach, (1995) R.A. Dixon and R. A. Gonzales, eds., IRL Press; and U.S. Patent No. 6,069,009.

Following preparation of the expression vector, the expression vector will be introduced into an appropriate host cell for production of the QC polypeptide, i.e. a host cell will be transformed with the expression vector. Introduction of the recombinant vector into a host cell is accomplished in any convenient manner, including, but not limited to, CaPO₄ precipitation, electroporation, microinjection, use of lipids (e.g., lipofectin), infection, and the like.

When any of the above host cells, or other appropriate host cells or organisms, are used to replicate and/or express the polynucleotides or nucleic acids of the invention, the resulting replicated nucleic acid, RNA, expressed protein or polypeptide, is within the scope of the invention as a product of the host cell or organism. The product is recovered by any appropriate means known in the art.

The subject nucleic acid molecules can be used to generate transgenic, non-human plants or animals or site-specific gene modifications in cell lines. Transgenic animals may be made through homologous recombination, where the endogenous locus is altered. Alternatively, a nucleic acid construct is randomly integrated into the genome. Vectors for stable integration include plasmids, retroviruses and other animal viruses, YACs, and the like. Transgenic insects are useful in screening assays, as described below. Insect transgenesis has been described in, e.g., "Insect Transgenesis: Methods and Applications" Handler and James, eds. (2000) CRC Press.

### ISOLATED POLYPEPTIDES OF THE INVENTION

The invention further provides isolated polypeptides comprising or consisting of an amino acid sequence of SEQ ID NO:02, or fragments, variants, or derivatives (e.g. orthologues) thereof. Compositions comprising any of these proteins may consist essentially of a subject protein, fragments, or derivatives, or may comprise additional components (*e.g.* pharmaceutically acceptable carriers or excipients, culture media, carriers used in pesticide formulations, *etc.*).

A derivative of a subject protein typically shares a certain degree of sequence identity or sequence similarity with SEQ ID NO:02, or a fragment thereof. As used herein, "percent (%) amino acid sequence identity" with respect to a subject sequence, or a specified portion of a subject sequence, is defined as the percentage of amino acids in the candidate derivative amino acid sequence identical with the amino acid in the subject sequence (or specified portion thereof), after aligning the sequences and introducing gaps, if necessary to achieve the maximum percent sequence identity, as generated by BLAST (Altschul *et al., supra*) using the same parameters discussed above for derivative nucleic acid sequences. A % amino acid sequence identity value is determined by the number of matching identical amino acids divided by the sequence length for which the percent identity is being reported.

"Percent (%) amino acid sequence similarity" is determined by doing the same calculation as for determining % amino acid sequence identity, but including conservative amino acid substitutions in addition to identical amino acids in the computation. A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine, and glycine.

In some embodiments, a subject protein variant or derivative shares at least about 60%, at least about 65%, at least about 70%, at least about 75%, at least 80% sequence identity or similarity, at least 85%, at least 90%, or at least about 95% sequence identity or similarity with a contiguous stretch of at least 25 amino acids, at least 50 amino acids, or at least 100 amino acids, and in some cases, the entire length of SEQ ID NO:02. In some embodiments, a polypeptide of the invention comprises an amino acid sequence as set forth in SEQ ID NO:02.

In some embodiments, a glutaminyl cyclase polypeptide of the invention comprises a fragment of at least about 6, at least about 10, at least about 15, at least about 20, at least about 25, at least about 30, at least about 40, at least about 50, at least about 75, at least about 100, at least about 125, at least about 150, at least about 175, at least about 200, at least about 225, at least about 250, at least about 275, at least about 300, at least about 325, or at least about 340, contiguous amino acids of the sequence set forth in SEQ ID NO:02. In many of these embodiments, the glutaminyl cyclase polypeptide has glutaminyl cyclase enzyme activity.

In many embodiments, a fragment or derivative of a subject protein is "functionally active" meaning that the subject protein derivative or fragment exhibits one or more functional activities associated with a full-length, wild-type subject protein comprising the amino acid sequence of SEQ ID NO:02. As one example, a fragment or derivative may have antigenicity such that it can be used in immunoassays, for immunization, for inhibition of activity of a subject protein, *etc,* as discussed further below regarding generation of antibodies to subject proteins. In many embodiments, a functionally active fragment or derivative of a subject protein is one that displays one or more biological activities associated with a subject protein, such as catalytic activity. For purposes herein, functionally active fragments also include those fragments that exhibit one or more structural features of a subject protein, such as transmembrane or enzymatic domains.

Protein domains can be identified using the PFAM program (Bateman A., et al., Nucleic Acids Res, 1999,27:260-2; http://pfam.wustl.edu). A glutaminyl cyclase domain, as identified by PFAM, is located at amino acids 46 to 344 of SEQ ID NO:02. Thus, an exemplary fragment consists of or comprises amino acids 46 to 346 of SEQ ID NO:02, and an exemplary derivative shares an above-described degree of identity with amino acids 46 to 346 of SEQ ID NO:02.

The functional activity of the subject proteins, derivatives and fragments can be assayed by various methods known to one skilled in the art (Current Protocols in Protein Science (1998) Coligan *et al.,* eds., John Wiley & Sons, Inc., Somerset, New Jersey). A spectrophtometric assay for cyclization of glutaminyl peptides to pyroglutamyl-peptides by glutaminyl cyclase has been described and may be used to assess whether a given polypeptide has glutaminyl cyclase activity. Bateman (1989) *J. Neurosci. Method* 30(1):23-28. In this assay, glutamate dehydrogenase catalyzes the reaction of NADH, α-ketoglutarate, and ammonia to yield NAD+ and glutamate. Reaction progress can be monitored by following a decrease in the absorbance of NADH at 340 nm. An HPLC assay for glutaminyl cyclase activity may also be used.

The subject proteins and polypeptides may be obtained from naturally occurring sources or synthetically produced. For example, wild type proteins may be derived from biological sources which express the proteins, e.g., *Heliothis.* The subject proteins may also be derived from synthetic means, e.g. by expressing a recombinant gene encoding protein of interest in a suitable host, as described above. Any convenient protein purification procedures may be employed, where suitable protein purification methodologies are described in Guide to Protein Purification, (Deuthser ed.) (Academic Press, 1990). For example, a lysate may prepared from the original source and purified using HPLC, exclusion chromatography, gel electrophoresis, affinity chromatography, and the like.

A derivative of a subject protein can be produced by various methods known in the art. The manipulations which result in their production can occur at the gene or protein level. For example, a cloned subject gene sequence can be cleaved at appropriate sites with restriction endonuclease(s) (Wells *et al*., Philos. Trans. R. Soc. London SerA (1986) 317:415), followed by further enzymatic modification if desired, isolated, and ligated *in vitro,* and expressed to produce the desired derivative. Alternatively, a subject gene can be mutated *in vitro* or *in vivo,* to create and/or destroy translation, initiation, and/or termination sequences, or to create variations in coding regions and/or to form new restriction endonuclease sites or destroy preexisting ones, to facilitate further *in vitro* modification. A variety of mutagenesis techniques are known in the art such as chemical mutagenesis, *in vitro* site-directed mutagenesis (Carter *et al.*, Nucl. Acids Res. (1986) 13:4331), use of TAB® linkers (available from Pharmacia and Upjohn, Kalamazoo, MI), *etc.*

At the protein level, manipulations include post translational modification, *e.g.* glycosylation, acetylation, phosphorylation, amidation, derivatization by known protecting/blocking groups, proteolytic cleavage, linkage to an antibody molecule or other cellular ligand, *etc.* Any of numerous chemical modifications may be carried out by known technique (*e.g.* specific chemical cleavage by cyanogen bromide, trypsin, chymotrypsin, papain, V8 protease, NaBH₄, acetylation, formylation, oxidation, reduction, metabolic synthesis in the presence of tunicamycin, *etc.*). Derivative proteins can also be chemically synthesized by use of a peptide synthesizer, for example to introduce nonclassical amino acids or chemical amino acid analogs as substitutions or additions into the subject protein sequence.

Chimeric or fusion proteins can be made comprising a subject protein or fragment thereof (e.g., comprising one or more structural or functional domains of the subject protein) joined at its amino- or carboxy-terminus via a peptide bond to an amino acid sequence of a different protein. Chimeric proteins can be produced by any known method, including: recombinant expression of a nucleic acid encoding the protein (comprising an amino acid sequence encoding a subject protein joined in-frame to a coding sequence for a different protein); ligating the appropriate nucleic acid sequences encoding the desired amino acid sequences to each other in the proper coding frame, and expressing the chimeric product; and protein synthetic techniques, *e.g.* by use of a peptide synthesizer.

### GENE REGULATORY ELEMENTS OF THE SUBJECT NUCLEIC ACID MOLECULES

The invention further provides gene regulatory DNA elements, such as enhancers or promoters that control transcription of the subject nucleic acid molecules. In some embodiments, a regulatory element resides within nucleotides 1-111 of SEQ ID NO: 1. Such regulatory elements can be used to identify tissues, cells, genes and factors that specifically control production of a subject protein. Analyzing components that are specific to a particular subject protein function can lead to an understanding of how to manipulate these regulatory processes, especially for pesticide and therapeutic applications, as well as an understanding of how to diagnose dysfunction in these processes.

Gene fusions with the subject regulatory elements can be made. For compact genes that have relatively few and small intervening sequences, such as those described herein for *Heliothis,* it is typically the case that the regulatory elements that control spatial and temporal expression patterns are found in the DNA immediately upstream of the coding region, extending to the nearest neighboring gene. Regulatory regions can be used to construct gene fusions where the regulatory DNAs are operably fused to a coding region for a reporter protein whose expression is easily detected, and these constructs are introduced as transgenes into the animal of choice. An entire regulatory DNA region can be used, or the regulatory region can be divided into smaller segments to identify sub-elements that might be specific for controlling expression a given cell type or stage of development. Reporter proteins that can be used for construction of these gene fusions include *E*. *coli* beta-galactosidase and green fluorescent protein (GFP). These can be detected readily *in situ,* and thus are useful for histological studies and can be used to sort cells that express a subject protein (O'Kane and Gehring PNAS (1987) 84(24):9123-9127; Chalfie *et al.*, Science (1994) 263:802-805; and Cumberledge and Krasnow (1994) Methods in Cell Biology 44:143-159). Recombinase proteins, such as FLP or cre, can be used in controlling gene expression through site-specific recombination (Golic and Lindquist (1989) Cell 59(3):499-509; White *et al.,* Science (1996) 271:805-807). Toxic proteins such as the reaper and hid cell death proteins, are useful to specifically ablate cells that normally express a subject protein in order to assess the physiological function of the cells (Kingston, In Current Protocols in Molecular Biology (1998) Ausubel *et al.,* John Wiley & Sons, Inc. sections 12.0.3-12.10) or any other protein where it is desired to examine the function this particular protein specifically in cells that synthesize a subject protein.

Alternatively, a binary reporter system can be used, similar to that described further below, where a subject regulatory element is operably fused to the coding region of an exogenous transcriptional activator protein, such as the GAL4 or tTA activators described below, to create a subject regulatory element "driver gene". For the other half of the binary system the exogenous activator controls a separate "target gene" containing a coding region of a reporter protein operably fused to a cognate regulatory element for the exogenous activator protein, such as UAS_{G} or a tTA-response element, respectively. An advantage of a binary system is that a single driver gene construct can be used to activate transcription from preconstructed target genes encoding different reporter proteins, each with its own uses as delineated above.

Subject regulatory element-reporter gene fusions are also useful for tests of genetic interactions, where the objective is to identify those genes that have a specific role in controlling the expression of subject genes, or promoting the growth and differentiation of the tissues that expresses a subject protein. Subject gene regulatory DNA elements are also useful in protein-DNA binding assays to identify gene regulatory proteins that control the expression of subject genes. The gene regulatory proteins can be detected using a variety of methods that probe specific protein-DNA interactions well known to those skilled in the art (Kingston, *supra)* including *in vivo* footprinting assays based on protection of DNA sequences from chemical and enzymatic modification within living or permeabilized cells; and *in vitro* footprinting assays based on protection of DNA sequences from chemical or enzymatic modification using protein extracts, nitrocellulose filter-binding assays and gel electrophoresis mobility shift assays using radioactively labeled regulatory DNA elements mixed with protein extracts. Candidate gene regulatory proteins can be purified using a combination of conventional and DNA-affinity purification techniques. Molecular cloning strategies can also be used to identify proteins that specifically bind subject gene regulatory DNA elements. For example, a *Drosophila* cDNA library in an expression vector, can be screened for cDNAs that encode subject gene regulatory element DNA-binding activity. Similarly, the yeast "one-hybrid" system can be used (Li and Herskowitz, Science (1993) 262:1870-1874; Luo *et al., Biotechniques* (1996) 20(4):564-568; Vidal *et al., Proc. Natl. Acad. Sci. USA* (1996) 93(19):10315-10320).

### ANTIBODIES SPECIFIC FOR SUBJECT PROTEINS

The present invention provides antibodies, which may be isolated antibodies, that bind specifically to a subject protein. The subject proteins, fragments thereof, and derivatives thereof may be used as an immunogen to generate monoclonal or polyclonal antibodies and antibody fragments or derivatives (*e.g*. chimeric, single chain, Fab fragments). As used herein, the term "antibodies" includes antibodies of any isotype, fragments of antibodies which retain specific binding to antigen, including, but not limited to, Fab, Fv, scFv, and Fd fragments, chimeric antibodies, humanized antibodies, single-chain antibodies, and fusion proteins comprising an antigen-binding portion of an antibody and a non-antibody protein. Also provided are "artificial" antibodies, e.g., antibodies and antibody fragments produced and selected *in vitro.* In some embodiments, such antibodies are displayed on the surface of a bacteriophage or other viral particle. In many embodiments, such artificial antibodies are present as fusion proteins with a viral or bacteriophage structural protein, including, but not limited to, M13 gene III protein. Methods of producing such artificial antibodies are well known in the art. See, e.g., U.S. Patent Nos. 5,516,637; 5,223,409; 5,658,727; 5,667,988; 5,498,538; 5,403,484; 5,571,698; and 5,625,033.

The antibodies may be detectably labeled, e.g., with a radioisotope, an enzyme which generates a detectable product, a green fluorescent protein, and the like. The antibodies may be further conjugated to other moieties, such as members of specific binding pairs, e.g., biotin (member of biotin-avidin specific binding pair), and the like. The antibodies may also be bound to a solid support, including, but not limited to, polystyrene plates or beads, and the like. For example, fragments of a subject protein, e.g., those identified as hydrophilic, are used as immunogens for antibody production using art-known methods such as by hybridomas; production of monoclonal antibodies in germ-free animals (PCT/US90/02545); the use of human hybridomas (Cole *et al*., *Proc. Natl*. *Acad. Sci. USA* (1983) 80:2026-2030; Cole *et al.*, in Monoclonal Antibodies and Cancer Therapy (1985) Alan R. Liss, pp. 77-96), and production of humanized antibodies (Jones *et al*., Nature (1986) 321:522-525; U.S. Pat. 5,530,101). In a particular embodiment, subject polypeptide fragments provide specific antigens and/or immunogens, especially when coupled to carrier proteins. For example, peptides are covalently coupled to keyhole limpet antigen (KLH) and the conjugate is emulsified in Freund's complete adjuvant. Laboratory animals, e.g., mice, rats, or rabbits are immunized according to conventional protocol and bled. The presence of specific antibodies is assayed by solid phase immunosorbent assays using immobilized corresponding polypeptide. Specific activity or function of the antibodies produced may be determined by convenient *in vitro,* cell-based, or *in vivo* assays: *e.g. in vitro* binding assays, *etc.* Binding affinity may be assayed by determination of equilibrium constants of antigen-antibody association (at least about 10⁷ M⁻¹, at least about 10⁸ M⁻¹, or at least about 10⁹ M⁻¹).

### SCREENING METHODS

A variety of methods can be used to identify or screen for molecules, such as proteins or other molecules, that interact with a subject protein, or derivatives or fragments thereof. The assays may employ purified protein, or cell lines or model organisms such as *Heliothis, Drosophila,* and *C. elegans,* that have been genetically engineered to express a subject protein. Suitable screening methodologies are well known in the art to test for proteins and other molecules that interact with a subject gene and protein (see *e.g.,* PCT International Publication No. WO 96/34099). The newly identified interacting molecules may provide new targets for pharmaceutical or pesticidal agents. Any of a variety of exogenous molecules, both naturally occurring and/or synthetic (*e.g.,* libraries of small molecules or peptides, or phage display libraries), may be screened for binding capacity. In a typical binding experiment, a subject protein or fragment is mixed with candidate molecules under conditions conducive to binding, sufficient time is allowed for any binding to occur, and assays are performed to test for bound complexes.

Assays to find interacting proteins can be performed by any method known in the art, for example, immunoprecipitation with an antibody that binds to the protein in a complex followed by analysis by size fractionation of the immunoprecipitated proteins (*e.g.* by denaturing or nondenaturing polyacrylamide gel electrophoresis), Western analysis, non-denaturing gel electrophoresis, two-hybrid systems (Fields and Song, Nature (1989) 340:245-246; U.S. Pat. NO. 5,283,173; for review see Brent and Finley, Annu. Rev. Genet. (1977) 31:663-704), *etc.*

### Immunoassays

Immunoassays can be used to identify proteins that interact with or bind to a subject protein. Various assays are available for testing the ability of a protein to bind to or compete with binding to a wild-type subject protein or for binding to an anti- subject protein antibody. Suitable assays include radioimmunoassays, ELISA (enzyme linked immunosorbent assay), immunoradiometric assays, gel diffusion precipitin reactions, immunodiffusion assays, *in situ* immunoassays (*e.g.,* using colloidal gold, enzyme or radioisotope labels), western blots, precipitation reactions, agglutination assays (*e.g.,* gel agglutination assays, hemagglutination assays), complement fixation assays, immunofluorescence assays, protein A assays, immunoelectrophoresis assays, *etc.*

One or more of the molecules in the immunoassay may be joined to a label, where the label can directly or indirectly provide a detectable signal. Various labels include radioisotopes, fluorescers, chemiluminescers, enzymes, specific binding molecules, particles, e.g. magnetic particles, and the like. Specific binding molecules include pairs, such as biotin and streptavidin, digoxin and antidigoxin etc. For the specific binding members, the complementary member would normally be labeled with a molecule that provides for detection, in accordance with known procedures.

### Identification of Potential Pesticide or Drug Targets

Once new target genes or target interacting genes are identified, they can be assessed as potential pesticide or drug targets, or as potential biopesticides. Further, transgenic plants that express subject proteins can be tested for activity against insect pests (Estruch *et al.,* Nat. Biotechnol (1997) 15(2):137-141).

The subject proteins are validated pesticide targets, since disruption in *Drosophila* of the subject genes results in lethality when homozygous. The mutation to lethality of these gene indicates that drugs that agonize or antagonize the gene product may be effective pesticidal agents.

As used herein, the term "pesticide" refers generally to chemicals, biological agents, and other compounds that adversely affect insect viability, e.g., that kill, paralyze, sterilize or otherwise disable pest species in the areas of agricultural crop protection, human and animal health. Exemplary pest species include parasites and disease vectors such as mosquitoes, fleas, ticks, parasitic nematodes, chiggers, mites, *etc.* Pest species also include those that are eradicated for aesthetic and hygienic purposes (*e.g.* ants, cockroaches, clothes moths, flour beetles, *etc.*), home and garden applications, and protection of structures (including wood boring pests such as termites, and marine surface fouling organisms).

Pesticidal compounds can include traditional small organic molecule pesticides (typified by compound classes such as the organophosphates, pyrethroids, carbamates, and organochlorines, benzoylureas, *etc.*). Other pesticides include proteinaceous toxins such as the *Bacillus thuringiensis* Crytoxins (Gill *et al.,* Annu Rev Entomol (1992) 37:615-636) and *Photorabdus luminescens* toxins (Bowden *et al.*, Science (1998) 280:2129-2132); and nucleic acids such as subject dsRNA or antisense nucleic acids that interfere with activity of a subject nucleic acid molecule.

The terms "candidate agent," "agent", "substance" and "compound" are used interchangeably herein. Candidate agents encompass numerous chemical classes, typically synthetic, semi-synthetic, or naturally-occurring inorganic or organic molecules. Candidate agents include those found in large libraries of synthetic or natural compounds. For example, synthetic compound libraries are commercially available from Maybridge Chemical Co. (Trevillet, Cornwall, UK), ComGenex (South San Francisco, CA), and MicroSource (New Milford, CT). A rare chemical library is available from Aldrich (Milwaukee, Wis.). Alternatively, libraries of natural compounds in the form of bacterial, fungal, plant and animal extracts are available from Pan Labs (Bothell, WA) or are readily producible.

Candidate agents may be small organic compounds having a molecular weight of more than 50 and less than about 2,500 daltons. Candidate agents may comprise functional groups necessary for structural interaction with proteins, particularly hydrogen bonding, and may include at least an amine, carbonyl, hydroxyl or carboxyl group, and may contain at least two of the functional chemical groups. The candidate agents may comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

Candidate agents that reduce a glutaminyl cyclase activity of a subject QC by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or more, are candidate pesticides.

Candidate agents that reduce glutaminyl cyclase activity of a subject glutaminyl cyclase are further tested for toxicity toward vertebrate species, such as mammalian species, etc.; for selectivity; and for bioavailability.

Pesticides can be delivered by a variety of means including direct application to pests or to their food source. In addition to direct application, toxic proteins and pesticidal nucleic acids (e.g. dsRNA) can be administered using biopesticidal methods, for example, by viral infection with nucleic acid or by transgenic plants that have been engineered to produce interfering nucleic acid sequences or encode the toxic protein, which are ingested by plant-eating pests.

Putative pesticides, drugs, and molecules can be applied onto whole insects, nematodes, and other small invertebrate metazoans, and the ability of the compounds to modulate (*e.g.* block or enhance) activity of a subject protein can be observed. Alternatively, the effect of various compounds on a subject protein can be assayed using cells that have been engineered to express one or more subject proteins and associated proteins.

A variety of other reagents may be included in the screening assay. These include reagents like salts, neutral proteins, e.g. albumin, detergents, etc that are used to facilitate optimal protein-protein binding and/or reduce non-specific or background interactions. Reagents that improve the efficiency of the assay, such as protease inhibitors, nuclease inhibitors, anti-microbial agents, etc. may be used. The components are added in any order that provides for the requisite activity. Incubations are performed at any suitable temperature, typically between 4°C and 40°C. Incubation periods are selected for optimum activity, but may also be optimized to facilitate rapid high-throughput screening. Typically between 0.1 and 1 hour will be sufficient.

### Assays of Compounds on purified QC

The invention provides methods of screening for agents that modulate an enzymatic activity of QC. Such agents are useful as pesticidal agents. An HPLC assay that directly monitors product formation by utilization of a dansylated peptide substrate has been described. This assay is useful for tracking activity in crude lysates and for validation of the HTS (high throughput screen) detection strategy. A second assay has been described which is based on an enzyme-coupled detection of the ammonia reaction product. In this reaction scheme, glutamate dehydrogenase catalyzes the reaction of NADH, α-ketoglutarate, and ammonia to yield NAD+ and glutamate. Reaction progress can be monitored by following a decrease in the absorbance of NADH at 340 nm. Such an assay is described in Example 2.

The present invention provides methods of identifying agents which modulate an enzymatic activity of a glutaminyl cyclase polypeptide of the invention. The term "modulate" encompasses an increase or a decrease in the measured glutaminyl cyclase activity when compared to a suitable control.

The method generally comprises: a) contacting a test agent with a sample containing a glutaminyl cyclase polypeptide; and b) assaying a glutaminyl cyclase activity of the glutaminyl cyclase polypeptide in the presence of the substance. An increase or a decrease in glutaminyl cyclase activity in comparison to glutaminyl cyclase activity in a suitable control (e.g., a sample comprising a glutaminyl cyclase polypeptide in the absence of the substance being tested) is an indication that the substance modulates an enzymatic activity of the glutaminyl cyclase.

An "agent which modulates a glutaminyl cyclase activity of a glutaminyl cyclase polypeptide", as used herein, describes any molecule, e.g. synthetic or natural organic or inorganic compound, protein or pharmaceutical, with the capability of altering a glutaminyl cyclase activity of a glutaminyl cyclase polypeptide, as described herein. Generally a plurality of assay mixtures is run in parallel with different agent concentrations to obtain a differential response to the various concentrations. Typically, one of these concentrations serves as a negative control, i.e. at zero concentration or below the level of detection.

### Assays of Compounds on Insects

Potential insecticidal compounds can be administered to insects in a variety of ways, including orally (including addition to synthetic diet, application to plants or prey to be consumed by the test organism), topically (including spraying, direct application of compound to animal, allowing animal to contact a treated surface), or by injection. Insecticides are typically very hydrophobic molecules and must commonly be dissolved in organic solvents, which are allowed to evaporate in the case of methanol or acetone, or at low concentrations can be included to facilitate uptake (ethanol, dimethyl sulfoxide).

The first step in an insect assay is usually the determination of the minimal lethal dose (MLD) on the insects after a chronic exposure to the compounds. The compounds are usually diluted in DMSO, and applied to the food surface bearing 0-48 hour old embryos and larvae. In addition to MLD, this step allows the determination of the fraction of eggs that hatch, behavior of the larvae, such as how they move /feed compared to untreated larvae, the fraction that survive to pupate, and the fraction that eclose (emergence of the adult insect from puparium). Based on these results more detailed assays with shorter exposure times may be designed, and larvae might be dissected to look for obvious morphological defects. Once the MLD is determined, more specific acute and chronic assays can be designed.

In a typical acute assay, compounds are applied to the food surface for embryos, larvae, or adults, and the animals are observed after 2 hours and after an overnight incubation. For application on embryos, defects in development and the percent that survive to adulthood are determined. For larvae, defects in behavior, locomotion, and molting may be observed. For application on adults, defects in levels and/or enzyme activity are observed, and effects on behavior and/or fertility are noted.

For a chronic exposure assay, adults are placed on vials containing the compounds for 48 hours, then transferred to a clean container and observed for fertility, defects in levels and/or activity of a subject enzyme, and death.

### Assay of Compounds using Cell Cultures

Compounds that modulate (*e.g.* block or enhance) a subject protein's activity may also be assayed using cell culture. Exemplary cells are cultured insect cells such as *Drosophila S2* cells. For example, various compounds added to cells expressing a subject protein may be screened for their ability to modulate the activity of subject genes based upon measurements of a biological activity of a subject protein. For example, compounds may be screened for their ability to modulate the activity of glutaminyl cyclase genes based on measurements of glutaminyl cyclase activity. Assays for changes in a biological activity of a subject protein can be performed on cultured cells expressing endogenous normal or mutant subject protein. Such studies also can be performed on cells transfected with vectors capable of expressing the subject protein, or functional domains of one of the subject protein, in normal or mutant form. In addition, to enhance the signal measured in such assays, cells may be cotransfected with nucleic acid molecules, or a subject recombinant vector, encoding a subject protein.

Alternatively, cells expressing a subject protein may be lysed, the subject protein purified, and tested *in vitro* using methods known in the art (Kanemaki M., et al., J Biol Chem, (1999) 274:22437-22444).

A wide variety of cell-based assays may be used for identifying agents which modulate levels of glutaminyl cyclase mRNA, for identifying agents that modulate the level of glutaminyl cyclase polypeptide, and for identifying agents that modulate the level of glutaminyl cyclase activity in a eukaryotic cell, using, for example, an insect cell (e.g., *Drosophila* S2 cells) transformed with a construct comprising a glutaminyl cyclase-encoding cDNA such that the cDNA is expressed, or, alternatively, a construct comprising a glutaminyl cyclase promoter operably linked to a reporter gene.

Accordingly, the present invention provides a method for identifying an agent, particularly a biologically active agent, that modulates a level of glutaminyl cyclase expression in a cell, the method comprising: combining a candidate agent to be tested with a cell comprising a nucleic acid which encodes a glutaminyl cyclase polypeptide; and determining the effect of said agent on glutaminyl cyclase expression (e.g., determining the effect of the agent on a level of glutaminyl cyclase mRNA, a level of glutaminyl cyclase polypeptide, or a level of glutaminyl cyclase enzyme activity in the cell).

"Modulation" of glutaminyl cyclase expression levels includes increasing the level and decreasing the level of glutaminyl cyclase mRNA and/or glutaminyl cyclase polypeptide encoded by the glutaminyl cyclase polynucleotide and/or the level of glutaminyl cyclase activity when compared to a control lacking the agent being tested. An increase or decrease of about 1.25-fold, usually at least about 1.5-fold, usually at least about 2-fold, usually at least about 5-fold, usually at least about 10-fold or more, in the level (i.e., an amount) of glutaminyl cyclase mRNA and/or polypeptide and/or QC enzyme activity following contacting the cell with a candidate agent being tested, compared to a control to which no agent is added, is an indication that the agent modulates glutaminyl cyclase mRNA levels, glutaminyl cyclase polypeptide levels, or glutaminyl cyclase enzyme activity in the cell. Of particular interest in many embodiments are candidate agents that reduce a level of glutaminyl cyclase mRNA, and/or reduce a level of glutaminyl cyclase polypeptide, and/or reduce a level of glutaminyl cyclase enzyme activity in an insect cell.

Glutaminyl cyclase mRNA and/or polypeptide whose levels or activitys are being measured can be encoded by an endogenous glutaminyl cyclase polynucleotide, or the glutaminyl cyclase polynucleotide can be one that is comprised within a recombinant vector and introduced into the cell, i.e., the glutaminyl cyclase mRNA and/or polypeptide can be encoded by an exogenous glutaminyl cyclase polynucleotide. For example, a recombinant vector may comprise an isolated glutaminyl cyclase transcriptional regulatory sequence, such as a promoter sequence, operably linked to a reporter gene (e.g,. β-galactosidase, CAT, luciferase, or other gene whose product can be easily assayed). In these embodiments, the method for identifying an agent that modulates a level of glutaminyl cyclase expression in a cell, comprises: combining a candidate agent to be tested with a cell comprising a nucleic acid which comprises a glutaminyl cyclase gene transcriptional regulatory element operably linked to a reporter gene; and determining the effect of said agent on reporter gene expression.

A recombinant vector may comprise an isolated glutaminyl cyclase transcriptional regulatory sequence, such as a promoter sequence, operably linked to sequences coding for a glutaminyl cyclase polypeptide; or the transcriptional control sequences can be operably linked to coding sequences for a glutaminyl cyclase fusion protein comprising glutaminyl cyclase polypeptide fused to a polypeptide which facilitates detection. In these embodiments, the method comprises combining a candidate agent to be tested with a cell comprising a nucleic acid which comprises a glutaminyl cyclase gene transcriptional regulatory element operably linked to a glutaminyl cyclase polypeptide-coding sequence; and determining the effect of said agent on glutaminyl cyclase expression, which determination can be carried out by measuring an amount of glutaminyl cyclase mRNA, glutaminyl cyclase polypeptide, glutaminyl cyclase fusion polypeptide, or glutaminyl cyclase enzyme activity produced by the cell.

Cell-based assays generally comprise the steps of contacting the cell with an agent to be tested, forming a test sample, and, after a suitable time, assessing the effect of the agent on glutaminyl cyclase mRNA levels, glutaminyl cyclase polypeptide and/or enzyme levels. A control sample comprises the same cell without the candidate agent added. Glutaminyl cyclase expression levels are measured in both the test sample and the control sample. A comparison is made between glutaminyl cyclase expression level in the test sample and the control sample. Glutaminyl cyclase expression can be assessed using conventional assays. For example, when a cell line is transformed with a construct that results in expression of glutaminyl cyclase, glutaminyl cyclase mRNA levels can be detected and measured, or glutaminyl cyclase polypeptide levels, and/or glutaminyl cyclase enzyme levels can be detected and measured. A suitable period of time for contacting the agent with the cell can be determined empirically, and is generally a time sufficient to allow entry of the agent into the cell and to allow the agent to have a measurable effect on glutaminyl cyclase mRNA and/or polypeptide levels and/or enzyme activity. Generally, a suitable time is between 10 minutes and 24 hours, more typically about 1-8 hours.

Methods of measuring glutaminyl cyclase mRNA levels are known in the art, several of which have been described above, and any of these methods can be used in the methods of the present invention to identify an agent which modulates glutaminyl cyclase mRNA level in a cell, including, but not limited to, a PCR, such as a PCR employing detectably labeled oligonucleotide primers, and any of a variety of hybridization assays. Similarly, glutaminyl cyclase polypeptide levels can be measured using any standard method, several of which have been described herein, including, but not limited to, an immunoassay such as ELISA, for example an ELISA employing a detectably labeled antibody specific for a glutaminyl cyclase polypeptide. Glutaminyl cyclase enzyme activity can be measured as described above.

Compounds that selectively modulate a level of a subject glutaminyl cyclase-encoding nucleic acid molecule, or that selectively modulate a level of a subject protein, or that selectively modulates a level of glutaminyl cyclase enzyme activity, are identified as potential pesticide and drug candidates having specificity for the subject protein. Whether a candidate compound selectively modulates a level of a subject glutaminyl cyclase-encoding nucleic acid molecule, or selectively modulates a level of a subject protein, or selectively modulates a level of glutaminyl cyclase enzyme activity can be determined by measuring the level of an mRNA or protein, e.g., GAPDH, or other suitable control protein or mRNA, where a candidate agent is "selective" if it does not substantially inhibit the production of or activity of any protein or mRNA other than a QC protein or QC-encoding mRNA.

Identification of small molecules and compounds as potential pesticides or pharmaceutical compounds from large chemical libraries requires high-throughput screening (HTS) methods (Bolger, Drug Discovery Today (1999) 4:251-253). Several of the assays mentioned herein can lend themselves to such screening methods. For example, cells or cell lines expressing wild type or mutant subject protein or its fragments, and a reporter gene can be subjected to compounds of interest, and depending on the reporter genes, interactions can be measured using a variety of methods such as color detection, fluorescence detection (*e.g.* GFP), autoradiography, scintillation analysis, *etc.*

Test agents that reduce QC activity can then be purified using conventional purification techniques, or can be synthesized *de novo* by conventional procedures.

Compounds identified using the above-described methods are useful to control pests, e.g., are useful as pesticides. Such compounds can control pests, e.g., by reducing pest growth, and/or fertility, and/or viability. The present invention provides compounds identified using any of the above-described assays. In many embodiments, an agent identified using the instant methods is purified, e.g., is separated from components (e.g., macromolecules and smaller compounds, e.g., down to about 50 Daltons) with which it is naturally associated, or, where the agent exists in a library, is separated from other members of the library. In many embodiments, a purified agent is at least about 50% pure, at least about 70% pure, at least about 80% pure, at least about 90% pure, at least about 95% pure, or at least about 99% pure.

Thus, in some embodiments, the invention provides a method for preparing a pesticidal agent that reduces enzymatic activity of an insect glutaminyl cyclase, generally involving identifying a candidate pesticidal agent, as described above; and purifying the agent. In general, a test agent that reduces glutaminyl cyclase activity of a subject QC by at least 20% when compared to a suitable control indicates that the test agent is a candidate pesticidal agent.

### PESTICIDAL AGENTS IDENTIFIED USING THE SUBJECT SCREENING METHODS

The present invention further provides pesticidal agents identified by a screening method of the invention.

Pesticides can be delivered by a variety of means including direct application to pests or to their food source. In addition to direct application, toxic proteins and pesticidal nucleic acids (e.g. dsRNA) can be administered using biopesticidal methods, for example, by viral infection with nucleic acid or by transgenic plants that have been engineered to produce interfering nucleic acid sequences or encode the toxic protein, which are ingested by plant-eating pests.

Putative pesticides, drugs, and molecules can be applied onto whole insects, nematodes, and other small invertebrate metazoans, and the ability of the compounds to modulate (*e.g.* block or enhance) activity of a subject protein can be observed. Alternatively, the effect of various compounds on a subject protein can be assayed using cells that have been engineered to express one or more subject proteins and associated proteins.

Of particular interest in many embodiments are agents that are selective inhibitors of an insect QC. "Selective inhibitors" are those agents that inhibit an insect QC but do not substantially inhibit a QC from a non-insect species.

A pesticidal composition of the invention comprises an agent that reduces the enzymatic activity of an insect QC by at least about 10%, at least about 20%, at least about 30%, at least about 40%, at least about 50%, at least about 60%, at least about 70%, or more. In particular, an agent inhibits enzymatic activity of an insect QC that has at least about 80%, at least about 85%, at least about 90%, at least about 95%, at least about 98%, or more amino acid sequence identity to SEQ ID NO:02. A subject pesticidal composition comprises an agent; and conventional excipients.

Agents that prove to be selective for invertebrate pests are formulated for application to an invertebrate pest population. Active agents can be formulated with an acceptable carrier into a pesticidal composition that is, for example, a suspension, a solution, an emulsion, a dusting powder, a dispersible granule, a wettable powder, an emulsifiable concentrate, an aerosol or impregnated granule. Formulations comprising an active agent identified by a screening method of the invention can be applied directly to plants to protect the plants against damage by an invertebrate pest, can be applied to the soil in which a plant to be protected is grown, or can be applied directly to the pest. Formulations for pesticides are well known in the art, and any known formulation can be used. U.S. Patent No. 6,180,088 describes foamable aerosol formulations for insecticidal compounds.

Such compositions disclosed above may be obtained by the addition of a surface active agent, an inert carrier, a preservative, a humectant, a feeding stimulant, an attractant, an encapsulating agent, a binder, an emulsifier, a dye, a U.V. protectant, a buffer, a flow agent, or other component to facilitate product handling and application for particular target pests.

Suitable surface-active agents include but are not limited to anionic compounds such as a carboxylate, for example, a metal carboxylate of a long chain fatty acid; an N-acylsarcosinate; mono or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate or dioctyl succinate.

Non-ionic surface active agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitar fatty acid esters, block copolymers of ethylene oxide and propylene oxide, acetylenic glycols such as 2,4,7,9-tetraethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such as an amine oxide of polyoxyethylene alkylamine; an amide-linked amine prepared by the condensation of a carboxylic acid with a di- or polyamine; or a quaternary ammonium salt.

Examples of inert materials include but are not limited to inorganic minerals such as kaolin, phyllosilicates, carbonates, sulfates, phosphates or botanical materials such as wood products, cork, powdered corncobs, peanut hulls, rice hulls, and walnut shells.

The compositions of the present invention can be in a suitable form for direct application or as a concentrate or primary powder which requires dilution with a suitable quantity of water or other diluent before application. The pesticidal concentration will vary depending upon the nature of the particular formulation, specifically, whether it is a concentrate or to be used directly. The composition generally contains 1 to 98% of a solid or liquid inert carrier, and 0 to 50%, or 0.1 to 50% of a surfactant. These compositions will be administered at the labeled rate for the commercial product, e.g., about 0.01 lb-5.0 lb per acre when in dry form and at about 0.01 pts-10 pts per acre when in liquid form.

### SUBJECT NUCLEIC ACIDS AS BIOPESTICIDES

Subject nucleic acids and fragments thereof, such as antisense sequences or double-stranded RNA (dsRNA), can be used to inhibit subject nucleic acid molecule function, and thus can be used as biopesticides. Methods of using dsRNA interference are described in published PCT application WO 99/32619. The biopesticides may comprise the nucleic acid molecule itself, an expression construct capable of expressing the nucleic acid, or organisms transfected with the expression construct. The biopesticides may be applied directly to plant parts or to soil surrounding the plants (e.g. to access plant parts growing beneath ground level), or directly onto the pest.

Biopesticides comprising a subject nucleic acid may be prepared in a suitable vector for delivery to a plant or animal. For generating plants that express the subject nucleic acids, suitable vectors include *Agrobacterium tumefaciens* Ti plasmid-based vectors (Horsch *et al.*, Science (1984) 233:496-89; Fraley *et al.*, Proc. Natl. Acad. Sci. USA (1983) 80:4803), and recombinant cauliflower mosaic virus (Hohn *et al*., 1982, In Molecular Biology of Plant Tumors, Academic Press, New York, pp 549-560; U.S. Patent No. 4,407,956 to Howell). Retrovirus based vectors are useful for the introduction of genes into vertebrate animals (Burns *et al.*, Proc. Natl. Acad. Sci. USA (1993) 90:8033-37).

Transgenic insects can be generated using a transgene comprising a subject gene operably fused to an appropriate inducible promoter. For example, a tTA-responsive promoter may be used in order to direct expression of a subject protein at an appropriate time in the life cycle of the insect. In this way, one may test efficacy as an insecticide in, for example, the larval phase of the life cycle (i.e. when feeding does the greatest damage to crops). Vectors for the introduction of genes into insects include P element (Rubin and Spradling, Science (1982) 218:348-53; U.S. Pat. No. 4,670,388), "hermes" (O'Brochta *et al*., Genetics (1996) 142:907-914), "minos" (U.S. Pat. No. 5,348,874), "mariner" (Robertson, Insect Physiol. (1995) 41:99-105), and "sleeping beauty"(Ivics *et al.*, Cell (1997) 91(4):501-510), "piggyBac" (Thibault *et al*., Insect Mol Biol (1999) 8(1):119-23), and "hobo" (Atkinson *et al*., Proc. Natl. Acad. Sci. U.S.A. (1993) 90:9693-9697). Recombinant virus systems for expression of toxic proteins in infected insect cells are well known and include Semliki Forest virus (DiCiommo and Bremner, J. Biol. Chem. (1998) 273:18060-66), recombinant sindbis virus (Higgs *et al*., Insect Mol. Biol. (1995) 4:97-103; Seabaugh *et al*., Virology (1998) 243:99-112), recombinant pantropic retrovirus (Matsubara *et al.*, Proc. Natl. Acad. Sci. USA (1996) 93:6181-85; Jordan *et al*., Insect Mol. Biol. (1998) 7:215-22), and recombinant baculovirus (Cory and Bishop, Mol. Biotechnol. (1997) 7(3):303-13; U.S. Patent No. 5,470,735; U.S. Patent Nos. 5,352,451; U.S. Patent No. 5, 770, 192; U.S. Patent No. 5,759,809; U.S. Patent No. 5,665,349; and U.S. Patent No. 5,554,592).

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

### Example 1: Cloning, sequencing, and characterization of Drosophila and Heliothis glutaminyl cyclase cDNAs

A Piggybac insertion strain in *Drosophila* (c04389) was identified and was observed to be lethal during the transition from the first to second larval instar. This insertion occurs in the 5'UTR, two nucleotides 5' of the initiating methionine, disrupting the translation initiation site around the ATG. The insertion is lethal in trans to a deficiency which uncovers the region in which the Piggybac is inserted. Excision of the Piggybac element confirmed that the lethality is due to the knockout of glutaminyl cyclase.

A *Heliothis* cDNA encoding the predicted QC protein was cloned and sequenced. The nucleotide and amino acid sequences are provided as SEQ ID NO:01 and 02, respectively. The amino acid sequence is provided in Figure 1; the nucleotide sequence is provided in Figure 2. BLAST analysis of the predicted protein sequence (SEQ ID NO:2) with sequences in GenBank reveals that the closest homolog is GenBank Identifier No. GI 7296321, which is described as a gene product from *Drosophila melanogaster,* and which shares 57% identity and 72% similarity with amino acids 33-339 of SEQ ID NO:2. The N-terminal two-thirds of the *Heliothis* QC-1 protein has 51% and 43% amino acid sequence identity to *Drosophila* and human sequences, respectively. A predicted signal peptide is consistent with cellular fractionation studies in which QC activity was observed to co-fractionate with secretory vesicles.

### Example 2: Spectrophotometric assay for glutaminyl cyclase

### Purification of Heliothis glutaminyl cyclase to use in assay

Amino acids 21-344 of *Heliothis* glutaminyl cyclase (QC) (SEQ ID NO:02) were fused in frame to an amino terminal glutathione S-tranferase/6-His epitope tag from the *E*. *coli* expression vector pET42b(+) (Novagen). The vector was introduced into *E*. *coli* and recombinant protein was expressed and purified using affinity chromatography.

### QC Assay

The assay, described in Bateman, *supra,* utilizes glutamate dehydrogenase, α-ketoglutarate, and NADH to measure the ammonia released during cyclization of the dipeptide substrate Gln-Gln. The amount of ammonia detected by this assay is proportional to the decrease in absorbance at 340 nm resulting from the conversion of NADH to NAD⁺.

The standard assay for QC includes saturating concentrations of α-ketoglutarate (13mM, Km=0.7 mM), NADH (0.65 mM, Km = 0.024 mM) and glutamate dehydrogenase (0.4 units), along with the aminopeptidase inhibitor arginine hydroxamate (0.65 mM), the substrate L-Gln-L-Gln (0.65 mM) and an appropriately diluted aliquot of a QC preparation, with or without a test agent (e.g., candidate pesticidal agent)in a final volume of 0.15 ml. The buffer used is 0.05 M N-Tris(hydroxymethyl) methyl-2-aminoethanesulfonic acid (TES), adjusted to pH 7.8, and containing 0.4 M NaCl. QC is added to initiate the reaction and the assay mixture is incubated for 1-2 hours at 37°C. The reaction is stopped by addition of 0.5 ml chilled water and the absorbance at 340 nm is recorded. Negative controls include either boiled enzyme or buffer substituted for native QC. The activity is quantitated by comparison of the recorded absorbance with a standard curve constructed with either ammonium sulfate or ammonium chloride. Assays are carried out under conditions of linear product formation.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. An isolated polynucleotide comprising a nucleotide sequence that encodes a polypeptide comprising the amino acid sequence set forth in SEQ ID NO:02.

2. An isolated polynucleotide comprising a nucleotide sequence having at least about 75% nucleotide sequence identity with the nucleotide sequence set forth in SEQ ID NO:01.

3. An isolated polynucleotide comprising a nucleotide sequence that hybridizes under stringent hybridization conditions to a nucleic acid molecule having the sequence set forth in SEQ ID NO:01.

4. A recombinant vector comprising a polynucleotide according to claim 1.

5. A recombinant host cell comprising a recombinant vector according to claim 4.

6. A method of producing an insect glutaminyl cyclase, comprising culturing the host cell of claim 5 under conditions suitable for expression of said protein and recovering said protein.

7. An isolated polypeptide comprising an amino acid sequence having at least about 80% sequence identity with the sequence set forth in SEQ ID NO:02.

8. A method for detecting an agent that reduces an enzymatic activity of an insect glutaminyl cyclase, said method comprising contacting said glutaminyl cyclase or fragment thereof having enzymatic activity with a test agent; and determining the effect, if any, of said test agent on glutaminyl cyclase activity of said enzyme or fragment; wherein the amino acid sequence of said glutaminyl cyclase comprises an amino acid sequence amino acid sequence which is at least about 80% identical to the sequence set forth in SEQ ID NO:02.

9. The method of Claim 8, further comprising selecting a test agent that reduces glutaminyl cyclase activity; determining an effect, if any, of the test agent on insect viabilitys, wherein a test agent that reduces insect viability is identified as a pesticidal agent.

10. The method of Claim 8 wherein said contacting comprises administering said test agent to cultured host cells that have been genetically engineered to produce said glutaminyl cyclase.

11. A method of controlling a pest, comprising contacting a pest with a compound identified by a method according to claim 8.

12. An isolated agent that reduces enzymatic activity of an insect glutaminyl cyclase.

13. A pesticidal composition comprising an agent that reduces enzymatic activity of an insect glutaminyl cyclase; and a carrier.

14. A method for preparing a pesticidal agent that reduces enzymatic activity of an insect glutaminyl cyclase, the method comprising:
contacting a test agent with an insect glutaminyl cyclase having at least about 80% amino acid sequence identity to the sequence set forth in SEQ ID NO:02;
determining the effect, if any, of said test agent on glutaminyl cyclase activity of said glutaminyl cyclase, wherein a reduction of glutaminyl cyclase activity of at least 20% when compared to a suitable control indicates that the test agent is a candidate pesticidal agent; and
purifying the candidate pesticidal agent.

15. A method of preparing a pesticidal composition, comprising:
combining an agent that reduces enzymatic activity of an insect glutaminyl cyclase; and an excipient.


SEQUENCE LISTING

<110> Ebens, Allen James Jr.
      Breach, Jean Claude R.
      Johnston, Stuart
      Mazzotta, Julie B.

<120> Polynucleotides Encoding Insect
  Glutaminyl Cyclase and Uses

<130> GNOP-006EP

<140> 60/293,369
<141> 2001-05-23

<160> 2

<170> FastSEQ for Windows Version 4.0

<210> 1
<211> 2235
<212> DNA
<213> Heliothis virescens

<400> 1
cagtgatatt gtgtgaagtg gccgaacgtg tgttacagta gcataggaaa tttgtactta      60
cgtttcgaaa gtcataaata acagcctgtc agttaataaa aaacattaaa tatgtttaaa     120
tcattactga atttggttgt tgtgatacta tgttttgcat ccgtaaacac aaaaaaacgg     180
ctaaagtttt acgaggaaaa gaacatccac gaggcacaag aactgaccga ccagaatgtg     240
agcgacttag cagcgttgtc agatatgaca catttcagaa aagtcctgga cgagatcctg     300
gtgccaaggg tagtgggcac cccaaaccat gacaaggtcg gcaactttat atcccaacaa     360
atgagggact taggctggga tgttactgag aatgttttct cagatacgac tcctatcttc     420
gggacgttga atttcaagaa tattattgcc aagttgaacc ctaatgctga aaggtttttg     480
gtcctcgcct gccattatga cagcaagtat atgagggagc atgtgtttgt tggtgcaacg     540
gattcagcag taccgtgcac catgatgata aacctggcca aagttatgtc agcccaactg     600
gatcctttga agaacaaccg gctgagtctc atgttcatat tctttgatgg cgaagaagcg     660
ttcaggcagt gggggcccaa tgactccatc tatggggccc ggcatttggc gaagaaatgg     720
cacggcacgg agtacaaaga tggcgcgaat gagctgcaga gaatggatgt cctagtcctc     780
ctcgacctgc taggcgctcc tgacccagtg ttcttcagct acttccaatc tacggagaaa     840
tggtacgtcc gcctagcagt ggccgagcag aggctggcgg agctgaacca gttcgaagcc     900
tactccaggg ggaaggtgga acagacgtac ttcaggctca ggagctccgg cgctgttata     960
gaagatgatc acataccgtt tatgaggaga aatgtggaca ttttacacgt gataccatcg    1020
ccgttcccgt cagtatggca cacgccagaa gacaatatgg cggcattaga cttcaaaact    1080
atagagaatc tcaacaaaat attcagagtt ttcgtcgccg agtaccttca tatacagccc    1140
aagtagcgga gatattaccc aggcaagata tttagttaat attgtaaatc tgaaaccgtt    1200
ttacgaaata ttgtatttgt agatagatga attttaatgt ttacgtacaa tttgtgaaat    1260
attatatgta ttgaaacaat gatttgacga atattcgccg atatgtatgg actttaaggc    1320
aggaacgaag tatctaagga aaatggagtc tatatatatt cgacctataa ttaattaatg    1380
ttcatttgat tatatgtgta ctcctatgta acgttgtcgg tgttcaataa ttcgctattt    1440
catattttaa ataagtactt atataaatgt tattatggtg aaaatgtaat ttccaaagta    1500
ttcttccaaa attatctatc tctctacatt cctttattat aagttgtgac caattaatta    1560
atttatatat ttatataaat atataggaaa aaatatatca gccgtgaagt gatattaaaa    1620
cataactaaa tgttttgtat aatatttaat tataaaagtt ttaataagtt acgtttcgtt    1680
tctgccaaat tgcacagaaa ccacgtgacc ctgcactcgg aagttagtag ctagccgaca    1740
atatgtttgg cgaaaactac atataggtat gtacaaacga caacacacaa gttaccacaa    1800
tctgtcaaat ttctgcgata gattttcaac cttatcacaa tagtggaagg ttaatattcg    1860
attggtaaaa tttgacaggt ttgggtagac tgacttgctg tcgactgtac ctacatagtt    1920
tacgaaacat caggtaaatg gtgttatttg ctctaatttt ccttaatatt tatattataa    1980
aattatcctt tttaatgtgt acattatatt tttatggatg attgattgtt atgttttggc    2040
ttttaattta atagttccag ttttgattaa tgaaattcga taagaaaatt agtataaatc    2100
aaaatcaaat cattttctat aatgttgtat atcatgctcg atgattcgcg aaaaaaacaa    2160
tttaattatt ttttgtctta taaatataat tttttgtttt gaattatttt acatttcctg    2220

aaaaaaaaaa aaaaa

<210> 2
<211> 344
<212> PRT
<213> Heliothis virescens

<400> 2
Met Phe Lys Ser Leu Leu Asn Leu Val Val Val Ile Leu Cys Phe Ala
1               5                   10                  15
Ser Val Asn Thr Lys Lys Arg Leu Lys Phe Tyr Glu Glu Lys Asn Ile
            20                  25                  30
His Glu Ala Gln Glu Leu Thr Asp Gln Asn Val Ser Asp Leu Ala Ala
        35                  40                  45
Leu Ser Asp Met Thr His Phe Arg Lys Val Leu Asp Glu Ile Leu Val
    50                  55                  60
Pro Arg Val Val Gly Thr Pro Asn His Asp Lys Val Gly Asn Phe Ile
65                  70                  75                  80
Ser Gln Gln Met Arg Asp Leu Gly Trp Asp Val Thr Glu Asn Val Phe
                85                  90                  95
Ser Asp Thr Thr Pro Ile Phe Gly Thr Leu Asn Phe Lys Asn Ile Ile
            100                 105                 110
Ala Lys Leu Asn Pro Asn Ala Glu Arg Phe Leu Val Leu Ala Cys His
        115                 120                 125
Tyr Asp Ser Lys Tyr Met Arg Glu His Val Phe Val Gly Ala Thr Asp
    130                 135                 140
Ser Ala Val Pro Cys Thr Met Met Ile Asn Leu Ala Lys Val Met Ser
145                 150                 155                 160
Ala Gln Leu Asp Pro Leu Lys Asn Asn Arg Leu Ser Leu Met Phe Ile
                165                 170                 175
Phe Phe Asp Gly Glu Glu Ala Phe Arg Gln Trp Gly Pro Asn Asp Ser
            180                 185                 190
Ile Tyr Gly Ala Arg His Leu Ala Lys Lys Trp His Gly Thr Glu Tyr
        195                 200                 205
Lys Asp Gly Ala Asn Glu Leu Gln Arg Met Asp Val Leu Val Leu Leu
    210                 215                 220
Asp Leu Leu Gly Ala Pro Asp Pro Val Phe Phe Ser Tyr Phe Gln Ser
225                 230                 235                 240
Thr Glu Lys Trp Tyr Val Arg Leu Ala Val Ala Glu Gln Arg Leu Ala
                245                 250                 255
Glu Leu Asn Gln Phe Glu Ala Tyr Ser Arg Gly Lys Val Glu Gln Thr
            260                 265                 270
Tyr Phe Arg Leu Arg Ser Ser Gly Ala Val Ile Glu Asp Asp His Ile
        275                 280                 285
Pro Phe Met Arg Arg Asn Val Asp Ile Leu His Val Ile Pro Ser Pro
    290                 295                 300
Phe Pro Ser Val Trp His Thr Pro Glu Asp Asn Met Ala Ala Leu Asp
305                 310                 315                 320
Phe Lys Thr Ile Glu Asn Leu Asn Lys Ile Phe Arg Val Phe Val Ala
                325                 330                 335
Glu Tyr Leu His Ile Gln Pro Lys
            340